# EUROPEAN PATENT SPECIFICATION

(11) **EP 1 765 274 B1**
(45) Date of publication and mention of the grant of the patent: **31.12.2008**
(21) Application number: 05750023.3
(22) Date of filing: 18.05.2005
(51) Int. Cl.: A61K 8/04, A61K 8/88, A61Q 17/02, A61Q 17/04, C08G 69/44

(54) **SKINCARE COMPOSITIONS**
HAUTPFLEGEZUSAMMENSETZUNGEN
COMPOSITIONS DE SOIN DE LA PEAU

(30) Priority: 14.06.2004 US 579735 P
(43) Date of publication of application: 28.03.2007
(73) Proprietor: SCHERING-PLOUGH HEALTHCARE PRODUCTS, INC., Memphis, TN 38151 (US)
(72) Inventor: FOWLER, Kevin, C., Millington, TN 38053 (US)
(74) Representative: UEXKÜLL & STOLBERG
(86) International application number: PCT/US2005/017256
(87) International publication number: WO 2006/001940

(56) References cited:
- WO-A-02/092663
- US-A1- 2003 198 613
- US-A1- 2003 223 943
- PATENT ABSTRACTS OF JAPAN vol. 1998, no. 02, 30 January 1998 (1998-01-30) & JP 09 263523 A (SHISEIDO CO LTD), 7 October 1997 (1997-10-07)

## Description

### BACKGROUND OF THE INVENTION

It is now generally recognized that exposure to solar radiation can have adverse health consequences, sometimes not appearing until several years following the exposure. Of course, the immediately appearing "sunburn" from an overexposure can itself be a serious acute health problem.

Many products are available to reduce the amount of solar ultraviolet radiation received by the skin during exposure to the sun's rays. Typical product formulations are lotions, creams, ointments or gels containing chemical and/or physical barriers to ultraviolet transmission. These vary considerably in their abilities to protect the skin against the physical and biochemical effects of ultraviolet radiation.

Earlier sunscreening formulations were designed to protect against sunburn from a limited solar exposure period, while transmitting sufficient radiation to permit skin tanning. However, the current focus is on eliminating as much ultraviolet exposure as possible, it being recognized that skin tanning, while esthetically pleasing to some, is a clear indication of tissue damage from overexposure to solar radiation. It has been recently discovered that any amount of unprotected exposure can potentially cause immune system suppression and lead to future health problems, such as skin carcinomas and other dermatological disorders.

The SPF (Sun Protection Factor) rating system has been developed to provide consumer guidance in selecting suitable sunscreens for any given outdoor activity. In general, the SPF number approximately corresponds to the multiple of time during which the properly applied sunscreen will prevent obvious reddening of the skin, over the exposure time that causes unprotected skin to exhibit reddening. Thus, if an SPF 8 sunscreen formulation has been properly applied, a person should be able to remain in the sun without visible effects for eight times the usual unprotected duration. Of course, the duration of unprotected exposure which produces a visible effect on the skin varies from one individual to another, due to differences in their skin cells. Currently popular are high-SPF "sunblocker" products, having SPF values of at least 30.

Most of the commercially available sunscreen formulations are not well suited for use by those engaged in strenuous outdoor activities, such as construction work, gardening, athletic events and many others, due to the tendency for perspiration from the body to interact with the applied formulation. For example, perspiration, or moisture from other sources, including rain, can cause sunscreen active ingredients and other irritating components of the formulation to enter the eyes and cause discomfort. It is also frequently detrimental, particularly in activities such as tennis or golf which require a reliable grip on equipment, to have an applied sunscreen formulation remain lubricious after application or become lubricious when mixed with perspiration or other moisture.

It is advantageous to have a suncare formulation that is waterproof. Waterproof formulations allow the user to engage in activities such as swimming while still being protected against ultraviolet radiation. Hydrophobic materials typically serve as waterproofing agents that impart film forming and waterproofing characteristics to an emulsion. However, there is still a need for products having physical attributes that display improved waterproof performance, and that have a reduction in migration of the formulation across the formulation wearer's skin, as well as providing a limited slip grip performance attribute.

A sunscreen product which has been available for several years, but which does not exhibit disadvantages such as the foregoing, is sold by Schering-Plough HealthCare Products, Inc., Memphis, Tenn. U.S.A. as COPPERTONE^{™} SPORT^{™} SPF 30 lotion. This product contains the active ingredients octyl salicylate, octyl methoxycinnamate, homosalate and oxybenzone, totaling 25.5 weight percent of the formulation, and is an oil-in-water emulsion formulated with 1.5 weight percent of a fumed silica having a hydrophobic surface treatment. It is thought that the silica serves to immobilize the active agents in the internal phase of the formulation and inhibit their migration under the influence of skin oils and/or external moisture. The product also has a very desirable "dry" feel as it is being applied, quite unlike the very liquid nature of the usual lotion which does not contain particulate ingredients other than those approved for use as sunscreen active ingredients.

There is a need for products having physical and performance attributes as those of the Coppertone Sport SPF 30 product, but which have more predictable formulation behavior and stability.

### SUMMARY OF THE INVENTION

Accordingly, there is disclosed an emulsion formulation for topical application to the skin comprising: an aqueous phase, an oil phase, at least one emulsifier, Ethylenediamine/NeopentylGlycol/Stearyl Hydrogenated Dimer and at least one sunscreen active agent.

There is also disclosed an emulsion formulation for topical application to the skin comprising an aqueous phase, an oil phase, at least one emulsifier, Ethylenediamine/NeopentylGlycol/Stearyl Hydrogenated Dimer Dilinoleate Copolymer, at least one sunscreen active agent and an insect repellant.

There is also disclosed an emulsion formulation for topical application to the skin comprising: an aqueous phase, an oil phase, at least one emulsifier, and an Ethylenediamine/NeopentylGlycol/Stearyl Hydrogenated Dimer Dilinoleate Copolymer, at least one sunscreen active agent and at least one skin protectant.

### DETAILED DESCRIPTION OF THE INVENTION

Names given to chemical substances herein generally are either accepted chemical names, or are trade organization or regulatory agency approved names such as CTFA Adopted Names as listed in J. A. Wenninger et al., Eds., CTFA International Cosmetic Ingredient Dictionary, Eighth Ed., The Cosmetic, Toiletry and Fragrance Association, Washington, D.C., 2000.

The term "percent by weight" as used herein means the percent by weight of the ingredient per weight of the overall formulation.

Suitable water proofing agents for use in the present invention include a material that is a dimerized C₁₈ polyamide resin. The INCI name for this material is Ethylenediamine/NeopentylGlycol/Stearyl Hydrogenated Dimer Dilinoleate Copolymer. The polyamide resin described herein results in formulas with a drier, more esthetically pleasing skin feel. In addition, formulas containing this material are not slippery on the hand, allowing a surer hand grip for the user. Unlike many other polyamide resins, this material has a softening point below 80 degrees Celsius which is an added convenience in formulating because the addition of the waterproofing agent allows the formulation to be formulated at lower temperatures and thus is more convenient. Another advantage of this ingredient is that it does not thicken formulas to the same degree as many other water resistance imparting agents, thus allowing more flexibility in viscosity control. Moreover, the use of this ingredient results in a composition that provides a degree of immediate water-proofing capability and thus obviates a waiting period before entering the water that is recommended with the use of conventional water-proof compositions. The material has been incorporated into an emulsion sunscreen composition and has been found to form a film which is substantive under running water. This water-proofing agent is sold under the trade name "Sylvaclear® C75V" and is available commercially from the Arizona Chemical Company of Lakeland, Florida. It may reportedly be prepared according to the procedures set forth in U.S. Patent No. 6,552,160,

The Ethylenediamine/NeopentylGlycol/Stearyl Hydrogenated Dimer Dilinoleate Copolymer may be present in an amount of about 0.05 percent to about 20 percent by weight, preferably about 1 percent to about 5 percent by weight, most preferably about 1 or 2 percent by weight.

The term "emulsion" shall be used herein to identify oil-in-water (o/w) or water-in-oil (w/o) type dispersion formulations intended for application to the skin, particularly lotions and creams providing cosmetic or therapeutic benefits. The emulsions may contain any of a number of desired "active" ingredients, including skin colorants, drug substances (such as antiinflammatory agents, antibiotics, topical anesthetics, antimycotics, keratolytics, etc.), skin protectants or conditioners, humectants, ultraviolet radiation absorbers, sunless tanning agents and the like, anti-oxidants, anti-aging agents, skin lightening agents, and insect repellants, depending on the intended uses for the formulations.

Techniques for forming o/w and w/o emulsions are very well known in the art. The present invention is not dependent upon any particular formulation technique, it being recognized that the choice of specific formulation components may well make necessary some specific formulation procedure.

Suitable emulsifiers for one aspect of the invention are those known in the art for producing oil-in-water and/or water-in-oil type emulsions. An aqueous external phase is preferred by many people for skin contact, since it is not as likely to produce an oily or greasy sensation when it is being applied, as is an emulsion having an oil external phase. The typical oil-in-water emulsifier has a hydrophilic-lipophilic balance (frequently abbreviated as "HLB") value greater than about 9, as is well known in the art; however, this "rule" is known to have numerous exceptions. The chosen emulsifier, depending upon its chemical nature, will be a component of either the oil or aqueous phase, and assists with both the formation and the maintenance, or stability, of the emulsion. Suitable emulsifiers for another aspect of the invention are those known in the art for producing water-in-oil type emulsions. The typical water-in-oil emulsifier has a HLB value of about 4 to about 6, as is well known in the art; however, this "rule" is also known to have numerous exceptions. Selection of suitable water-in-oil emulsifiers is well known in the formulation art.

Most of the widely used emulsifier systems for sunscreen formulations can be used in the invention. Particularly preferred emulsifiers are PEG-8 Distearate available under the trade name of Emerest 2712 from Henkel, PEG-5 Glyceryl Stearate available under the trade name POEM-S-105 from Riken Vitamin Oil, PEG-6 Hydrogenated Castor Oil, available under the trade name Sabowax ELH6 from Sabo, PEG-6 Oleate, available under the trade name STEPAN PEG-300 MO from Stepan, Sorbitan Sesquioleate, available under the trade name Arlacel 83 and Arlacel C from ICI Surfactants, TEA-Stearate, available under the trade name of Cetasal from Gattefosse S.A. Another preferred emulsifier is neutralized cetyl phosphate, available under the trade name Amphisol A from LaRoche. Most preferred is an Acrylate/C₁₀-C₃₀ alkyl acrylate cross polymer of C₁₀₋₃₀ alkyl acrylates and one or more monomers of acrylic acid, methacrylic acid or one of their simple esters crosslinked within allyl ether of sucrose or an allyl ether of pentaerythritol, available under the trade names of Pemulen TR-1 and Pemulen TR-2 from B.F. Goodrich. The amount of emulsifier used in the present invention is present in an amount of about 0.1 to about 10% by weight, preferably about 0.5 percent to about 5 percent by weight, most preferably about 2 percent to about 4 percent by weight. The choice of an emulsifier is well within ordinary skill in the art and is not a critical aspect of the invention. Additional preferred emulsifiers that may be employed include Sorbitan Triisostearate available under the trade name Crill 6 from Croda Oleochemicals, and Polyglyceryl-3 Distearate available under the trade name Cremophor GS 32 from BASF.

As is known in the art, it is preferred that the individual emulsion droplets have a small and uniform size because these properties result in a more stable emulsion. Conversely, a broad particle size distribution indicates that the interfacial tension between the droplets has not been substantially reduced, and thus the droplets tend to coalesce and form agglomerations that result in an unstable emulsion.

The formulations of the present invention provide an elegant feel upon application to the skin, while also possessing an improved ability to withstand high temperature and shear during formulation, as well as, improved stability over time relative to other skin care products currently manufactured.

For purposes of the present invention, a "sunscreen active agent" shall include all of those materials, singly or in combination, that are regarded as acceptable for use as active sunscreening ingredients. Approval by a regulatory agency is generally required for inclusion of active agents in formulations intended for human contact, and those active agents which have been or are currently approved for sunscreen use in the United States include, without limitation, para aminobenzoic acid, avobenzone, cinoxate, dioxybenzone, homosalate, menthyl anthranilate, octocrylene, octyl methoxycinnamate, octyl salicylate, oxybenzone, padimate O, phenylbenzimidazole sulfonic acid, sulisobenzone, trolamine salicylate, titanium dioxide, zinc oxide, diethanolamine methoxycinnamate, digalloy trioleate, ethyl dihydroxypropyl PABA, glyceryl aminobenzoate, lawsone with dihydroxyacetone, red petrolatum.

Particularly preferred sunscreen active agents include homomenthyl salicylate available under the trade name Uniderm Homsal from Universal Preserv-A-Chem, Benzophenone-3, available under the trade name Escalol 567 from ISP VanDyk, Uvinul MS-40 from BASF and Uvasorb MET/C from 3V Inc., Octyl Salicylate available under the trade name Neo Heliopan OS from Haarmann & Reimer, Octocrylene available under the trade name Uvinul N-539-SG from BASF and the trade name Neo Heliopan 303 from Haarmann & Reimer, and Octyl methoxcinnamate, available under the trade name Parsol MCX from Givaudon Roure and LaRoche, or mixtures thereof.

It is typical to use combinations of two or more sunscreen ingredients in a formulation, to achieve higher levels of ultraviolet absorption or to provide useful absorption over a wider range of ultraviolet wavelengths than can be the case with a single active component. Several other sunscreen active ingredients are accepted for use in other countries, such as terephthalylidene dicamphor sulfonic acid, sold under the trademark of Mexoryl® SX, and these are also considered to be within the scope of the present invention.

The formulations of the present invention will have either sunscreen capability (SPF <30) and/or sunblock capability (SPF >30). Preferably, the formulations of the present invention will have an SPF greater than at least about 30, preferably greater than at least about 40, and preferably about 50.

Insect repelling components are desirable in sunscreening emulsions, since the emulsions are normally used primarily by persons engaged in outdoor activities. The most widely used active agent for personal care products is N,N-Diethyl-m-toluamide, frequently called "DEET" and available in the form of a concentrate containing at least about 95 percent DEET. Other synthetic chemical repellents include dimethyl phthalate, ethyl hexanediol, indalone, di-n-propylisocinchoronate, bicycloheptene, dicarboximide and tetrahydrofuraldehyde. Certain plant-derived materials also have insect repellent activity, including citronella oil and other sources of citronella (including lemon grass oil), limonene, rosemary oil and eucalyptus oil. Choice of an insect repellent for incorporation into the sunscreen emulsion will frequently be influenced by the odor of the repellent. The amount of repellent agent used will depend upon the choice of agent; DEET is useful at high concentrations, such as up to about 15 percent or more, while some of the plant-derived substances are typically used in much lower amounts, such as 0.1 percent or less.

As used herein, an after sun emulsion formulation is defined as a formulation that can be administered after a user has been in the sun for any amount of time that provides a soothing or healing effect that is pleasant to the user. Such a formulation can contain, for instance, aloe vera, vitamins A, C and E, greetn tea extract, etc.

Also within the scope of the present invention are skin protectant active agents. Suitable examples include, among others: (a) Allantoin, 0.5 to 2 percent; (b) Aluminum hydroxide gel, 0.15 to 5 percent; (c) Calamine, 1 to 25 percent; (d) Cocoa butter, greater than 50; (e) Cod liver oil, 5 to 13.56 percent; (f) Colloidal oatmeal; (g) Dimethicone, 1 to 30 percent; (h) Glycerin, 20 to 45 percent; (i) Hard fat, greater than 50; (j) Kaolin, 4 to 20 percent; (k) Lanolin, 12.5 to 50 percent; (I) Mineral oil, greater than 50 percent; (m) Petrolatum, greater than 30 percent; (n) Sodium bicarbonate; (o) Topical starch, 10 to 98 percent; (p) White petrolatum, greater than 30 percent; (q) Zinc acetate, 0.1 to 2 percent; (r) Zinc carbonate, 0.2 to 2 percent; and (s) Zinc oxide, 1 to 25 percent.

The compositions of the present invention may contain a wide range of additional, optional components. The CTFA Cosmetic Ingredient Handbook, Seventh Edition, 1997 and the Eighth Edition, 2000, which is incorporated by reference herein in its entirety, describes a wide variety of cosmetic and pharmaceutical ingredients commonly used in skin care compositions, which are suitable for use in the compositions of the present invention. Examples of these functional classes disclosed in this reference include: absorbents, abrasives, anticaking agents, antifoaming agents, antioxidants, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, plasticizers, preservatives, propellants, reducing agents, skin bleaching agents, skin-conditioning agents (emollient, humectants, miscellaneous, and occlusive), skin protectants, solvents, foam boosters, hydrotropes, solubilizing agents, suspending agents (nonsurfactant), sunscreen agents, ultraviolet light absorbers, waterproofing agents, and viscosity increasing agents (aqueous and nonaqueous).

Water is employed in amounts effective to form the emulsion. It is generally preferred to use water which has been purified by processes such as deionization or reverse osmosis, to improve the batch-to-batch formulation inconsistencies which can be caused by dissolved solids in the water supply. The amount of water in the emulsion or composition can range from about 15 percent to 95 weight percent, preferably from about 45 to 75 percent, most preferably from about 60 percent to about 75 percent.

Alternatively, in another embodiment of the present invention, the ingredients may be dissolved in a monohydric alcohol solvent. The present invention may contain up to 90% of monohydric alcohol(s). The preferred amount of the monohydric alcohol is about 25% to about 85% and most preferably from about 35% to about 75%. In this embodiment, the sunscreen active agent or agents may be present in an amount of about 25% to about 30% and the waterproofing agent may be present in an amount of about 1& to about 5%. In a still further embodiment of the present invention, the formulation may be a monohydric alcohol based gel formulation.

An emollient is an oleaginous or oily substance which helps to smooth and soften the skin, and may also reduce its roughness, cracking or irritation. Typical suitable emollients include mineral oil having a viscosity in the range of 50 to 500 centipoise (cps), lanolin oil, coconut oil, cocoa butter, olive oil, almond oil, macadamia nut oil, aloe extracts such as aloe vera lipoquinone, synthetic jojoba oils, natural sonora jojoba oils, safflower oil, corn oil, liquid lanolin, cottonseed oil and peanut oil. Preferably, the emollient is a cocoglyceride, which is a mixture of mono, di and triglycerides of cocoa oil, sold under the trade name of Myritol 331 from Henkel KGaA, or Dicaprylyl Ether available under the trade name Cetiol OE from Henkel KGaA or a C₁₂-C₁₅ Alkyl Benzoate sold under the trade name Finsolv TN from Finetex. One or more emollients may be present ranging in amounts from about 1 percent to about 10 percent by weight, preferably about 5 percent by weight. Another suitable emollient is DC 200 Fluid 350, a silicone fluid, available Dow Corning Corp.

Other suitable emollients include squalane, castor oil, polybutene, sweet almond oil, avocado oil, calophyllum oil, ricin oil, vitamin E acetate, olive oil, silicone oils such as dimethylopolysiloxane and cyclomethicone, linolenic alcohol, oleyl alcohol, the oil of cereal germs such as the oil of wheat germ, isopropyl palmitate, octyl palmitate, isopropyl myristate, hexadecyl stearate, butyl stearate, decyl oleate, acetyl glycerides, the octanoates and benzoates of (C₁₂ -C₁₅) alcohols, the octanoates and decanoates of alcohols and polyalcohols such as those of glycol and glyceryl, ricinoleates esters such as isopropyl adipate, hexyl laurate and octyl dodecanoate, dicaprylyl maleate, hydrogenated vegetable oil, phenyltrimethicone, jojoba oil and aloe vera extract.

Other suitable emollients which are solids or semi-solids at ambient temperatures may be used. Such solid or semi-solid cosmetic emollients include glyceryl dilaurate, hydrogenated lanolin, hydroxylated lanolin, acetylated lanolin, petrolatum, isopropyl lanolate, butyl myristate, cetyl myristate, myristyl myristate, myristyl lactate, cetyl alcohol, isostearyl alcohol and isocetyl lanolate. One or more emollients can optionally be included in the formulation.

A humectant is a moistening agent that promotes retention of water due to its hygroscopic properties. Suitable humectants include glycerin, polymeric glycols such as polyethylene glycol and polypropylene glycol, mannitol and sorbitol. Preferably, the humectant is Sorbitol, 70% USP or polyethylene glycol 400, NF. One or more humectants can optionally be included in the formulation in amounts from about 1 percent to about 10 percent by weight, preferably about 5 percent by weight.

A dry-feel modifier is an agent which when added to an emulsion, imparts a "dry feel" to the skin when the emulsion dries. Dry feel modifiers can include talc, kaolin, chalk, zinc oxide, silicone fluids, inorganic salts such as barium sulfate, surface treated silica, precipitated silica, fumed silica such as an Aerosil available from Degussa Inc. of New York, N.Y. U.S.A. Another dry feel modifier is an epichlorohydrin cross-linked glyceryl starch of the type that is disclosed in U.S. Patent No. 6,488,916, issued in the name of Fowler and assigned to Schering-Plough Healthcare Products, Inc.

It may be advantageous to incorporate additional thickening agents, such as, for instance, Carbopol Ultrez, or alternatively, Carbopol ETD 2001, available from the B. F. Goodrich Co. The selection of additional thickening agents is well within the skill of one in the art.

An additional waterproofing or water resistance agent is a hydrophobic material that imparts film forming and waterproofing characteristics to an emulsion. A suitable additional waterproofing agent is a copolymer of vinyl pyrollidone and eicosene and dodecane monomers such as Ganex V 220 and Ganex V 216 Polymers, respectively, trade names of ISP Inc. of Wayne, N.J. U.S.A. Still other suitable waterproofing agents include polyethylene polymer, such as Performa V 825 available from New Phase Technologies and polyanhydride resin No. 18 available under the trade name PA-18 from Chevron. The waterproofing agent is used in amounts effective to allow the sunscreen to remain effective on the skin after exposure to circulating water for at least 40 minutes for water resistance and at least 80 minutes for waterproofing using the procedures described by the U.S. Food and Drug Administration in "Sunscreen Drug Products for OTC Human Use," Federal Register, Vol. 43, Aug. 25, 1978, Part 2, pp. 38206-38269.

An antimicrobial preservative is a substance or preparation which destroys, or prevents or inhibits the proliferation of, microorganisms in the sunscreen composition, and which may also offer protection from oxidation. Preservatives are frequently used to make self-sterilizing, aqueous based products such as emulsions. This is done to prevent the development of microorganisms that may be in the product from growing during manufacturing and distribution of the product and during use by consumers, who may further inadvertently contaminate the products during normal use. Typical preservatives include the lower alkyl esters of para-hydroxybenzoates (parabens), especially methylparaben, propylparaben, isobutylparaben and mixtures thereof, benzyl alcohol, phenyl ethyl alcohol and benzoic acid, diazolydinyl, urea, chlorphenesin, iodopropynyl and butyl carbamate. The preferred preservative is available under the trade name of Germaben II from Sutton. One or more antimicrobial preservatives can optionally be included in an amount ranging from about 0.001 to about 10 weight percent, preferably about 0.05 to about 1 percent.

An antioxidant is a natural or synthetic substance added to the sunscreen to protect from or delay its deterioration due to the action of oxygen in the air (oxidation). They may also reduce oxidation reactions in skin tissueAnti-oxidants prevent oxidative deterioration which may lead to the generation of rancidity and nonenyzymatic browning reaction products. Typical suitable antioxidants include propyl, octyl and dodecyl esters of gallic acid, butylated hydroxyanisole (BHA, usually purchased as a mixture of ortho and meta isomers), butylated hydroxytoluene (BHT), green tea extract, uric acid, cysteine, pyruvate, nordihydroguaiaretic acid, Vitamin A, Vitamin E and Vitamin C and their derivatives. One or more antioxidants can optionally be included in the sunscreen composition in an amount ranging from about 0.001 to about 5 weight percent, preferably about 0.01 to about 0.5 percent.

Chelating agents are substances used to chelate or bind metallic ions, such as with a heterocylic ring structure so that the ion is held by chemical bonds from each of the participating rings. Suitable chelating agents include ethylene diaminetetraacetic acid (EDTA), EDTA disodium, calcium disodium edetate, EDTA trisodium, albumin, transferrin, desferoxamine, desferal, desferoxamine mesylate, EDTA tetrasodium and EDTA dipotassium. One or more chelating agents can optionally be included in the sunscreen in amounts ranging from about 0.001 to about 0.5 weight percent preferably about 0.01 % weight percent.

Fragrances are aromatic substances which can impart an aesthetically pleasing aroma to the sunscreen composition. Typical fragrances include aromatic materials extracted from botanical sources (i.e., rose petals, gardenia blossoms, jasmine flowers, etc.) which can be used alone or in any combination to create essential oils. Alternatively, alcoholic extracts may be prepared for compounding fragrances. However, due to the relatively high costs of obtaining fragrances from natural substances, the modern trend is to use synthetically prepared fragrances, particularly in high-volume products. The preferred fragrances for use in the present invention are Fragrance SZ-2108 and Fragrance SZ-1405 available from Sozio, Inc... One or more fragrances can optionally be included in the sunscreen composition in an amount ranging from about 0.001 to about 5 weight percent, preferably about 0.01 to about 0.5 percent by weight.

A pH modifier is a compound that will adjust the pH of a formulation to a lower, e.g., more acidic pH value, or to a higher, e.g., more basic pH value. The selection of a suitable pH modifier is well within the ordinary skill of one in the art.

The invention will be further described by means of the following examples, which are not intended to limit the invention, as defined by the appended claims, in any manner.

### EXAMPLE 1

| **Percent W/W** | **Ingredient Description** |
|---|---|
| **Part A** | |
| 47.7 | USP Purified Water |
| 0.38 | Pemulen TR-1 |
| 5 | Propylene Glycol USP |
| 0.01 | Disodium EDTA |
| 0.5 | Allantoin |
| 0.5 | Benzyl Alcohol |
| 0.2 | Methylparaben, NF |

| **Part B** | |
|---|---|
| 4 | Lexfeel 7, |
| 7.5 | Octinoxate, USP |
| 6 | Oxybenzone, USP |
| 0.01 | Vitamin E, DL Alpha Tocopherol |
| 0.2 | Oleth-3 |
| 5 | Octisalate, USP |
| 0.1 | Propylparaben NF |
| 9 | Homomenthyl Salicylate; Homosal |
| 1.5 | Sylvaclear C75V |
| 2 | Octocrylene |

| **Part C** | |
|---|---|
| 10 | USP Purified Water |
| 0.35 | Triethanolamine 99% NF |

| **Part D** | |
|---|---|
| 0.05 | Fragrance SZ-2108 |

The ingredients of part A were mixed with the exception of the Pemulen and allantoin. The Pemulen was slowly sprinkled in and then the allantoin was mixed in by stirring rapidly. The ingredients of part b were mixed in a second beaker and heated to about 175 degrees Celsius. The two phases were then mixed with a disperser for about 15 minutes. Triethanolomine and water were then added. The fragrance was then added with stirring for about 2 minutes.

### EXAMPLE 2

| **Percent W/W** | **Ingredient Description** |
|---|---|
| **Part A** | |
| 48 | USP Purified Water |
| 0.38 | Pemulen TR-1 |
| 5 | Propylene Glycol USP |
| 0.01 | Disodium EDTA |
| 1 | Germaben |

| **Part B** | |
|---|---|
| 7.5 | Octinoxate, USP |
| 6 | Oxybenzone, USP |
| 0.01 | Vitamin E, DL Alpha Tocopherol |
| 0.2 | Oleth-3 |
| 5 | Octisalate, USP |
| 13 | Homomenthyl Salicylate; Homosal |
| 1.5 | Sylvaclear C75V |
| 2 | Octocrylene |

| **Part C** | |
|---|---|
| 10 | USP Purified Water |
| 0.35 | Triethanolamine 99% NF |

| **Part D** | |
|---|---|
| 0.05 | Fragrance SZ-2108 |

The ingredients of part A were mixed with the exception of the Pemulen. The Pemulen was slowly sprinkled in. The ingredients of part B were mixed in a second beaker and heated to about 175 degrees Celsius. The two phases were then mixed with a disperser for about 15 minutes. Triethanolomine and water were then added. The fragrance was then added with stirring for about 2 minutes.

The sunscreen lotion prepared in accord with the procedures set forth in this example produces a high SPF sun block with an SPF of about 50.

## Claims

1. An emulsion formulation for topical application to the skin comprising: an aqueous phase, an oil phase, at least one emulsifier, an Ethylenediamine/NeopentylGlycol/Stearyl Hydrogenated Dimer Dilinoleate Copolymer and at least one sunscreen active agent.

2. The formulation of claim 1, wherein the formulation is an oil-in water emulsion.

3. The formulation of claim 2, wherein the at least one emulsifier is present in an amount of 1 to 10 percent by weight.

4. The formulation of claim 1, wherein the formulation is a water-in-oil emulsion.

5. The formulation of claim 4, wherein the at least one emulsifier is present in an amount of 1 to 10 percent by weight.

6. The formulation of claim 1, wherein the Ethylenediamine/NeopentylGlycol/Stearyl Hydrogenated Dimer Dilinoleate Copolymer is present in an amount of 0.1 to 20 percent by weight.

7. The formulation of claim 1, wherein the formulation has an SPF of at least 30.

8. The formulation of claim 1, wherein the formulation has an SPF of at least 40.

9. The formulation of claim 1, further comprising at least one skin protectant.

10. The formulation of claim 1, further comprising at least one insect repellant.

## Patentansprüche

1. Emulsionsformulierung zur topischen Aufbringung auf die Haut, welche eine wässrige Phase, eine Ölphase, mindestens einen Emulgator, ein Ethylendiamin/Neopentylglykol/Stearyl-hydriertes Dimer-Dilinoleat-Copolymer und mindestens ein als Sonnenschutz wirkendes Mittel enthält.

2. Formulierung nach Anspruch 1, die eine Öl-in-Wasser-Emulsion ist.

3. Formulierung nach Anspruch 2, bei der der mindestens eine Emulgator in einer Menge von 1 bis 10 Gew.-% vorhanden ist.

4. Formulierung nach Anspruch 1, die eine Wasser-in-Öl-Emulsion ist.

5. Formulierung nach Anspruch 4, bei der der mindestens eine Emulgator in einer Menge von 1 bis 10 Gew.-% vorhanden ist.

6. Formulierung nach Anspruch 1, bei der das Ethylendiamin/Neopentylglykol/Stearyl-hydrierte Dimer-Dilinoleat-Copolymer in einer Menge von 0,1 bis 20 Gew.-% vorhanden ist.

7. Formulierung nach Anspruch 1, bei der die Formulierung einen Lichtschutzfaktor (SPF) von mindestens 30 aufweist.

8. Formulierung nach Anspruch 1, bei der die Formulierung einen Lichtschutzfaktor (SPF) von mindestens 40 aufweist.

9. Formulierung nach Anspruch 1, die ferner mindestens ein Hautschutzmittel enthält.

10. Formulierung nach Anspruch 1, die ferner mindestens ein Mittel zur Abwehr von Insekten enthält.

## Revendications

1. Formulation en émulsion pour application en topique sur la peau, comprenant : une phase aqueuse, une phase huileuse, au moins un émulsifiant, un copolymère éthylènediamine/néopentylglycol/(dimère dilinoléate de stéaryle hydrogéné), et au moins un agent à action d'écran solaire.

2. Formulation conforme à la revendication 1, laquelle formulation est une émulsion huile-dans-eau.

3. Formulation conforme à la revendication 2, dans laquelle ledit émulsifiant au nombre d'au moins un se trouve présent en une proportion pondérale de 1 à 10 %.

4. Formulation conforme à la revendication 1, laquelle formulation est une émulsion eau-dans-huile.

5. Formulation conforme à la revendication 4, dans laquelle ledit émulsifiant au nombre d'au moins un se trouve présent en une proportion pondérale de 1 à 10 %.

6. Formulation conforme à la revendication 1, dans laquelle le copolymère éthylènediamine/néopentylglycol/(dimère dilinoléate de stéaryle hydrogéné) se trouve présent en une proportion pondérale de 0,1 à 20 %.

7. Formulation conforme à la revendication 1, qui offre un indice de protection SPF d'au moins 30.

8. Formulation conforme à la revendication 1, qui offre un indice de protection SPF d'au moins 40.

9. Formulation conforme à la revendication 1, qui comprend en outre au moins un agent de protection de la peau.

10. Formulation conforme à la revendication 1, qui comprend en outre au moins un agent répulsif anti-insectes.
